# EUROPEAN PATENT SPECIFICATION

(11) **EP 4 710 338 B1**
(45) Date of publication and mention of the grant of the patent: **04.11.2026**
(21) Application number: 23729887.2
(22) Date of filing: 09.05.2023
(51) Int. Cl.: G16H 20/17, A61M 5/142, A61M 5/172, G16H 40/20, G16H 40/63, G16H 40/67, A61B 5/00

(54) **DEVICE, SYSTEM, AND METHOD FOR PREDICTING UPCOMING INFUSION ALARM AND NOTIFYING CLINICIAN OF THE SAME**
VORRICHTUNG, SYSTEM UND VERFAHREN ZUR VORHERSAGE EINES BEVORSTEHENDEN INFUSIONSALARMS UND BENACHRICHTIGUNG DES ARZTES DAMIT
DISPOSITIF, SYSTÈME ET PROCÉDÉ DE PRÉDICTION D'ALARME DE PERFUSION À VENIR ET DE NOTIFICATION DE CELLE-CI À UN CLINICIEN

(43) Date of publication of application: 18.03.2026
(73) Proprietor: CareFusion 303, Inc., San Diego, CA 92130 (US)
(72) Inventor: WORKMAN, Michael K., San Diego, California 92130 (US); LANGAN, John, San Diego, California 92130 (US)
(74) Representative: Zenz Patentanwälte Partnerschaft mbB
(86) International application number: PCT/US2023/021577
(87) International publication number: WO 2024/232873

(56) References cited:
- JP-A- 2005 237 666
- US-A1- 2009 156 975
- US-A1- 2010 010 330
- US-A1- 2019 274 624

## Description

### BACKGROUND

Many patient care devices include alarms for communicating urgent information to clinicians. These alarms are useful, but their prevalence in healthcare environments can lead to "alarm fatigue" for clinicians. Alarm fatigue occurs when clinicians become desensitized to the alarms and, as a result, fail to respond appropriately. Additionally, the alarms are often jarring for patients trying to rest and recover.

US 2019/274624 A1 describes systems, methods, and devices for generating alarms and alerts within an on-body diabetes management network. The system receives glucose data from a continuous glucose monitor and determines insulin dosage accordingly. It detects alarm or alert conditions and transmits wireless notifications of these events to a remote user interface device. If the alert is not acknowledged within a defined time frame, the insulin delivery device activates an audible, visual, or haptic alarm to notify the user.

US 2010/010330 A1 relates to a monitor device for a fluid infusion system, particularly for insulin infusion. The device wirelessly receives data from components such as an infusion pump or glucose sensor transmitter and presents the status through graphical and alarm features. It can display indicators of remaining operational resources, such as reservoir volume or battery life, and estimate future physiological measurements of the patient based on empirical data.

US 2009/156975 A1 concerns systems for blood withdrawal and reinfusion for measurement purposes. The described systems and methods manage operational issues such as occlusions or bubbles in the blood access line, performing automatic cleaning and determining the patency of the access site. The system can identify trends suggesting occlusions, recognize the operational phase-such as withdrawal, infusion, or cleaning-and locate problems to enable corrective procedures.

JP 2005/237666 A describes a system for monitoring and displaying the progress of intravenous transfusions or medication effects. A nurse call terminal receives input of medical orders and transfusion start time. Based on these inputs, it predicts multiple progress milestones and the completion time, updating display patterns accordingly.

### SUMMARY

The invention is defined by the subject matter of the independent claims, while preferred embodiments and further developments are set forth in the dependent claims.

According to various aspects of the subject technology, an infusion device includes an infusion pump, a sensor, an alarm, and a processor. The infusion pump is configured to deliver a fluid in accordance with an infusion therapy. The sensor is configured to generate sensor data for an infusion metric associated with the infusion therapy. The alarm is configured to trigger when the sensor data indicates that the infusion metric satisfies a safety threshold. And the processor is configured to monitor the sensor data generated by the sensor. The processor is also configured to determine, based on the sensor data, a predicted alarm time at which the infusion metric is predicted to satisfy the safety threshold. Additionally, the processor is configured to determine, based on a type of the alarm, a type of the fluid, or a care area at which the infusion device is located, a lead time threshold before the predicted alarm time. Further, the processor is configured to, based on a current time being within the lead time threshold, and prior to the predicted alarm time and without triggering the alarm, provide a notification regarding the alarm.

According to various aspects of the subject technology, a computer-implemented method for managing alarms of an infusion device includes monitoring sensor data generated by a sensor of an infusion device, the sensor being configured to generate the sensor data for an infusion metric associated with an infusion therapy. The method also includes determining, based on the sensor data, a predicted alarm time at which the infusion metric is predicted to satisfy a safety threshold, where an alarm of the infusion device is configured to trigger when the sensor data indicates that the infusion metric satisfies the safety threshold. Additionally, the method includes determining, based on a type of the alarm, a type of a fluid administered by an infusion pump of the infusion device in accordance with the infusion therapy, or a care area at which the infusion device is located, a lead time threshold before the predicted alarm time. Further, the method includes, based on a current time being within the lead time threshold, and prior to the predicted alarm time and without triggering the alarm, providing a notification regarding the alarm.

According to various aspects of the subject technology, a non-transitory, machine-readable medium embodies instructions that, when executed by a machine, facilitate the machine to perform any of the computer-implemented method discussed herein, such as the aforenoted computer-implemented method.

It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

For a better understanding of the various described implementations, reference should be made to the Detailed Description below, in conjunction with the Figures. Like reference numerals refer to corresponding parts throughout the Figures and the Description.
FIGS. 1A and 1B depict an example patient care system that includes infusion pumps mounted to a control unit, according to various aspects of the subject technology.
FIG. 2 depicts an example institutional patient care system of a healthcare organization, according to various aspects of the subject technology.
FIG. 3 depicts example clinician response times to an infusion alarm, according to various aspects of the subject technology
FIGS. 4A and 4B depict example pre-alarm notifications displayed at a control unit and a mobile device, respectively, according to various aspects of the subject technology.
FIG. 5 depicts an example process for managing alarms of an infusion device, according to various aspects of the subject technology.
FIG. 6 is a conceptual diagram illustrating an example electronic system for managing alarms of an infusion device, according to various aspects of the subject technology.

### DETAILED DESCRIPTION

Reference will now be made to implementations, examples of which are illustrated in the accompanying drawings. In the following description, numerous specific details are set forth in order to provide an understanding of the various described implementations. However, it will be apparent to one of ordinary skill in the art that the various described implementations may be practiced without these specific details. In some instances, well-known methods, procedures, components, circuits, and networks have not been described in detail so as not to unnecessarily obscure aspects of the implementations.

The present disclosure is directed to managing alarms for an infusion device. Alarms can consume valuable resources of an infusion device. For example, some infusion devices may include visual or audio alarms that can be activated when certain conditions are detected. Activation of these devices can consume resources (e.g., power, processing time, network traffic, network interface, etc.) of the infusion device that might otherwise be used to deliver fluids or other operations.

Furthermore, some alarms may interrupt operation of the infusion device. The process of interrupting, confirming a restart, and restarting the infusion device can also consume resources. For example, there may be momentum built up in a cam used to drive the pump that, if stopped, would be lost. In losing the momentum, the pump may need extra cycles or other resources to return to the pre-pause operational efficiency. This introduces potential loss due to the alarm. Stopping and restarting an infusion may also impact characteristics of the flow because the tubing or other pumping elements may relax or change temperature after a pause.

In addition to these technical advantages, the features discussed herein may also alleviate alarm fatigue and deeilcrease patient disturbance caused by infusion alarms in the healthcare environment. As discussed herein, these and other benefits can be achieved by predicting upcoming infusion alarms and acting before the alarms are triggered. For example, when a clinician has advance notice of an upcoming alarm, the clinician can respond to and resolve the alarm condition, thus avoiding the alarm and/or eliminating (or at least minimizing) the conditions leading to the alarm.

As discussed herein, the subject technology may be implemented in an infusion device that includes an alarm configured to trigger when an infusion metric satisfies a safety threshold. The infusion metric may, for example, be an internal infusion metric (e.g., infusion runtime) or an infusion metric detected by a sensor of the infusion device. According to various implementations, the infusion device is configured to determine a predicted alarm time and a lead time threshold (e.g., a threshold amount of time) before the predicted alarm time for alerting a clinician of the upcoming alarm. The infusion device then provides a notification of the alarm to a clinician within the lead time threshold and before the alarm is triggered.

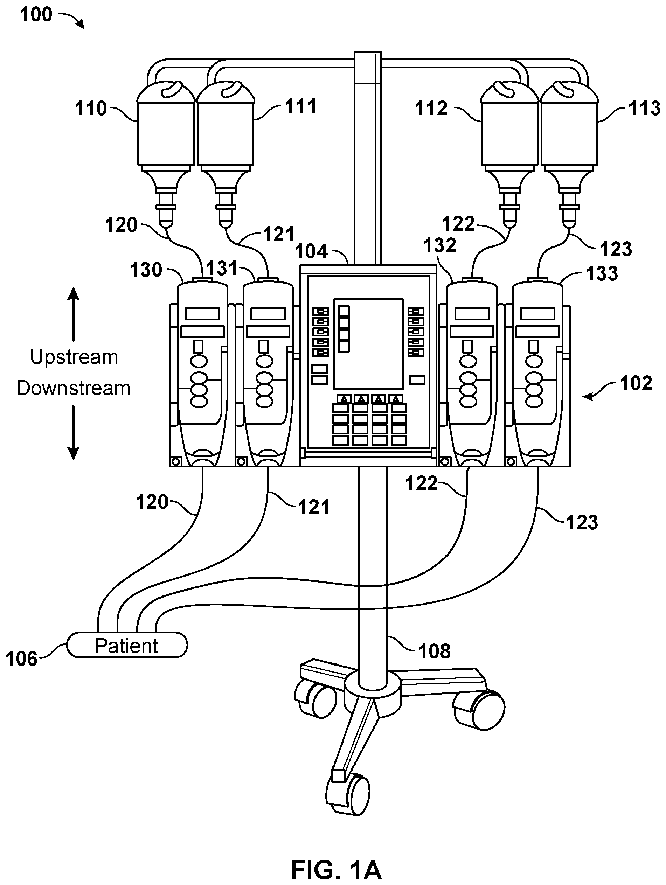

FIG. 1A depicts an example patient care system 100 that includes four infusion pumps 130-133 mounted to a control unit 104, according to various aspects of the subject technology. Each of the infusion pumps 130-133 is in operative engagement with a respective administration set 120-123 (e.g., silicon tubing). The administration sets 120-123 connect the infusion pumps 130-133 to fluid supplies 110-113, which are inverted and suspended above the infusion pumps 130-133. The fluid supplies 110-113 are depicted as bottles in FIG. 1A, but they may take other forms (e.g., bags). Both the fluid supplies 110-113 and the patient care device 102 are mounted to a roller stand 108.

The fluid supplies 110-113, as well as their orientation (e.g., mount location, mount height, mounting type, etc.) within the care area, may generate one or more interaction records. The interaction record for a set, for example, may be generated in part by detecting a scannable code associated with the set or by detecting a physical structure on the set that encodes identifying information for the set prior to use.

As shown in FIG. 1A, each administration set 120-123 is connected between a respective fluid supply 110-113 and a patient 106 so that the patient 106 may receive the fluids in any or all the fluid supplies 110-113. Each of the administration sets 120-123 may be identified either actively (e.g., by clinician scanning) or passively (e.g., by wireless or optical detection).

In the depicted example, a separate infusion pump 130-133 is used to infuse each of the respective fluids of the fluid supplies 110-113 into the patient 106. The infusion pumps 130-133 are flow control devices that will act on a fluid conduit of the respective administration set 120-123 to move fluid from the fluid supply 110-113, through the fluid conduit, and to the patient. Because individual infusion pumps 130-133 are used, each of the infusion pumps 130-133 may be individually set to the pumping or operating parameters required for infusing the particular medical fluid from the respective fluid supply 110-113 into the patient at the particular rate prescribed for that fluid by the clinician.

Typically, medical administration sets have more parts than are shown in FIG. 1A. Many have check valves, drip chambers, valved ports, connectors, and other devices well known to those skilled in the art. These other devices are not included in FIG. 1A to preserve clarity of illustration.

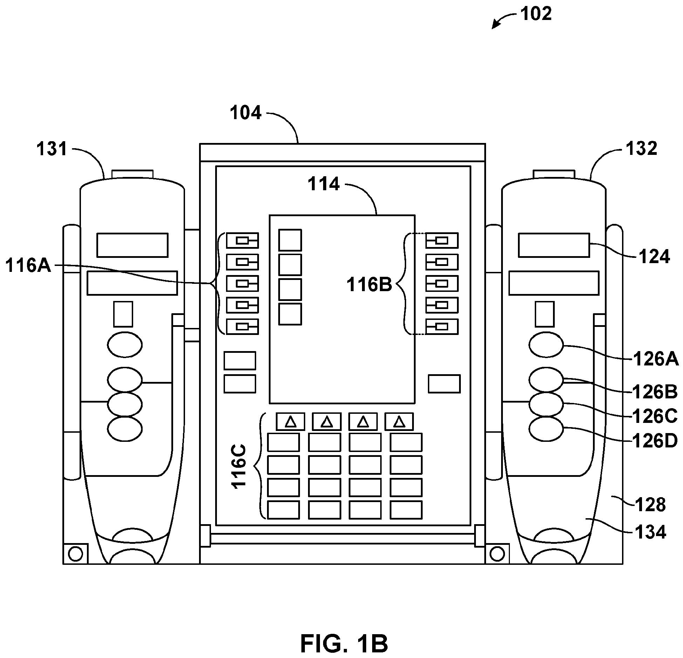

FIG. 1B depicts a closer view of a portion of the example patient care system 100 shown in FIG. 1A, according to various aspects of the subject technology. FIG. 1B depicts the control unit 104, with two of the infusion pumps 131-132 mounted at either side of the control unit 104 (also referred to herein as an infusion control unit). In some implementations, the control unit 104 is configured for programming each of the infusion pumps 130-133. FIG. 1B also shows the displays and controls of the infusion pumps 131-132, such as display 124 and controls 126A-D of infusion pump 132.

Each of the infusion pumps 130-133 includes a door and a handle. For example, as depicted in FIG. 1B, infusion pump 132 includes a door 128 and a handle 134. The handle 134 operates to lock the door 128 in a closed position during operation. The handle 134 also operates to unlock and open the door 128 for loading an administration set (e.g., administration set 122) and for accessing the internal pumping and sensing mechanisms of the infusion pump 132. While the door 128 is open, the administration set can be connected with the pump 132. While the door 128 is closed, the administration set is brought into operative engagement with the pumping mechanism, upstream and downstream pressure sensors, and/or other equipment of the infusion pump 132.

The display 124 of the infusion pump 132 (e.g., an LED display) is located in on the door 128 in the depicted embodiment and may be used to visually communicate information regarding the infusion pump 132. For example, the display 124 can communicate alert indications, such as an alert regarding an upcoming or currently active alarm. Additionally, the control keys 126A-D exist for programming and controlling operations of the infusion pump as desired. In some implementations, the control keys may be presented as interactive elements on the display 124 (e.g., a touchscreen display). The patient care device 102 and/or infusion pump 132 may also include audio alert equipment in the form of a speaker.

The control unit 104 of the patient care device 102 includes a display 114 for visually communicating various information, such as the operating parameters of a connected pump or alert indications and alert messages. The control unit 104 also includes control keys 116A-C for selecting or setting control parameters and/or options for controlling the control unit 104 and modules connected thereto (e.g., infusion pumps 130-133).

In addition to the display 114 and the control keys 116A-C, the control unit 104 may also include a speaker to provide audible alerts. In some implementations, the display 114 is implemented as a touchscreen display. In such implementations, the control keys 116A-C may be omitted or reduced in number by providing corresponding interactive elements via a graphical user interface presented via the display 114. In some implementations, the control keys 116A-C may select a corresponding option displayed in display 114.

The control unit 104 may include a communication module (not depicted) by which the control unit 104 can communicate with external equipment, such as a medical facility server, a handheld communication device, a laptop-type computer, or other information device that a clinician may have to transfer information as well as to download drug libraries to the control unit.

The communication module may be used to transfer access or interaction information for clinicians encountering the control unit 104 or a device coupled thereto (e.g., the infusion pumps 130-133, or a bar code scanner). The communications system may include one or more of a radio frequency (RF) system, an optical system (e.g., an infrared system), a BLUETOOTH^{™} system, or another wired or wireless system.

The bar code scanner and communications system may alternatively be included integrally with the infusion pumps 130-133, such as in cases where a control unit is not used, or in addition to one with the control unit 104. Further, information input devices need not be hard-wired to medical instruments, information may be transferred through a wireless connection as well. Additionally, other types of modules may be connected to the infusion pumps 130-133 or to the control unit 104 such as a syringe pump module, patient controlled analgesic module, end-tidal CO₂ monitoring module, oximeter monitoring module, or the like.

In some embodiments, pressure measurements sensors (e.g., a pressure sensor) in the infusion pumps 130-133 are transmitted to a server or other coordination device, and the methods disclosed herein are implemented on the server or another coordination device. For example, more sophisticated and computationally intensive approaches like machine-learning can be implemented on the server or on a patient care device with a larger memory or processing resources. In some embodiments, machine learning is used to identify empty conditions based on pressure signals received from the pump.

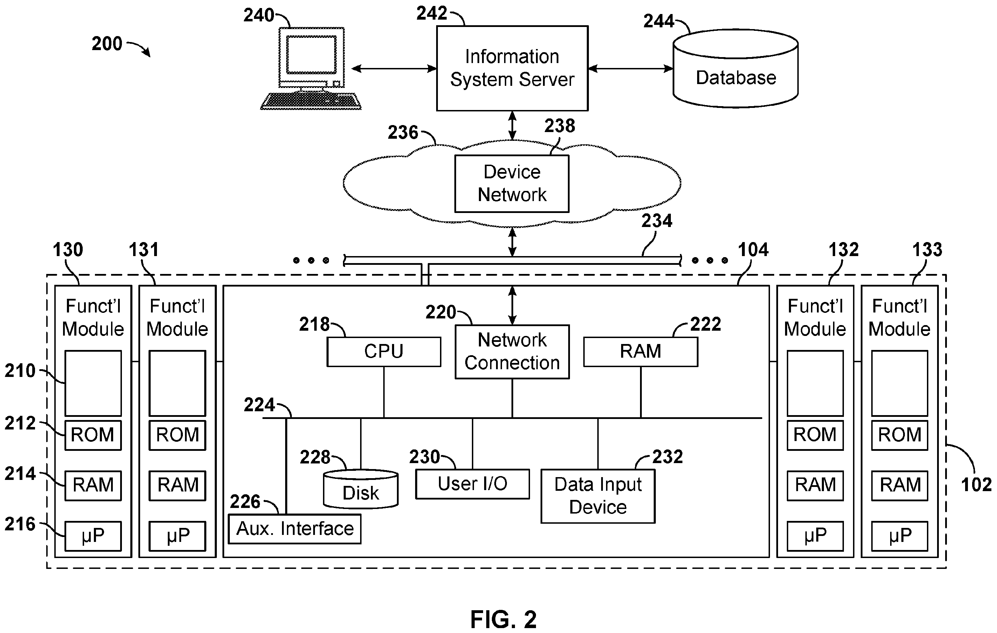

FIG. 2 depicts an example institutional patient care system 200 of a healthcare organization, according to various aspects of the subject technology. In FIG. 2, a patient care device 102 is connected to an internal healthcare network 236. As used herein, the terms "patient care device" or "PCD" may be used interchangeably with the term patient care unit, and "PCU," either of which may include various ancillary medical devices such as an infusion pump (e.g., the infusion pumps 130-133 of FIG. 1A), a vital signs monitor, a medication dispensing device (e.g., cabinet, tote), a medication preparation device, an automated dispensing device, a module coupled with one of the aforementioned (e.g., a syringe pump module coupled with an infusion pump), or other similar devices. Each element of the patient care device 102 is connected to an internal healthcare network 236 by a transmission channel 234. The transmission channel 234 may be any wired or wireless transmission channel, such as an 802.11 wireless local-area-network (WLAN).

In some implementations, the internal healthcare network 236 also includes computer systems located in various departments throughout a hospital or healthcare center. For example, the internal healthcare network 236 optionally includes computer systems associated with an admissions department, a billing department, a biomedical engineering department, a clinical laboratory, a central supply department, one or more unit station computers, and/or a medical decision support system. As described further below, the internal healthcare network 236 may include discrete subnetworks. For instance, in the depicted example, the internal healthcare network 236 includes a device network 238 by which the patient care device 102 may communicate with other devices in accordance with normal operations.

The institutional patient care system 200 may also include an information system server 242, such as a health information system (HIS) server. Moreover, although the information system server 242 is shown as a separate server, the functions and programming of the information system server 242 may be incorporated into another computer.

Additionally, the institutional patient care system 200 may include a device terminal 240 for connecting and communicating with the information system server 242. The device terminal 240 may include personal computers, personal data assistants, and mobile devices, such as laptops, tablet computers, augmented reality devices, or smartphones, configured with software for communicating with the information system server 242 via the internal healthcare network 236.

The patient care device 102 includes a system for providing patient care, and may include or incorporate pumps (e.g., infusion pumps 130-133 of FIG. 1A), physiological monitors (e.g., heart rate, blood pressure, ECG, EEG, pulse oximeter, and other patient monitors), therapy devices, and other drug delivery devices may be utilized according to the teachings set forth herein.

In the depicted example, the patient care device 102 comprises a control unit 104, connected to one or more functional modules 130-133 (e.g., the infusion pumps 130-133 of FIG. 1A). The control unit 104 includes a central processing unit (CPU) 218 connected to a memory, for example, random access memory (RAM) 222, and one or more interface devices such as user interface device 230, a data input device 232, a network connection 220, and an auxiliary interface 226 for communicating with additional modules or devices. The control unit 104 also, although not necessarily, includes a main non-volatile storage unit 228, such as a hard disk drive or non-volatile flash memory, for storing software data. Additionally, the control unit 104 may include one or more internal buses 224 for interconnecting the aforementioned elements.

In various implementations, the user interface device 230 includes a touch screen for displaying information to a user and allowing a user to input information by touching defined areas of the screen. Additionally, or in the alternative, the user interface device 230 could include any means for displaying and inputting information, such as a monitor, a printer, a keyboard, softkeys, a mouse, a track ball, and/or a light pen.

The data input device 232 may be a bar code reader capable of scanning and interpreting data printed in bar coded format. Additionally, or in the alternative, the data input device 232 can be any device for entering coded data into a computer, such as a device for reading magnetic strips, radio-frequency identification (RFID) devices whereby digital data encoded in RFID tags or smart labels (defined below) are captured by the data input device 232 via radio waves, PCMCIA smart cards, radio frequency cards, memory sticks, CDs, DVDs, or any other analog or digital storage media. Other examples of the data input device 232 include a voice activation or recognition device or a portable personal data assistant (PDA).

Depending upon the types of interface devices used, the user interface device 230 and the data input device 232 may be the same device. Although the data input device 232 is shown in FIG. 2 as being disposed within the control unit 104, it is recognized that the data input device 232 may be external to the control unit 104 (e.g., at the device terminal 240).

The auxiliary interface 226 may be an RS232 interface; however, any other means for communicating with a peripheral device (e.g., a printer, a patient monitor, an infusion pump, or another medical device) may be used without departing from the subject technology. Additionally, the data input device 232 may be a separate functional module (e.g., one of functional modules 130-133) configured to communicate with the control unit 104 or any other system on the network using suitable programming and communication protocols.

The network connection 220 may be a wired or wireless connection, such as via Ethernet, WiFi, BLUETOOTH, an integrated services digital network (ISDN) connection, a digital subscriber line (DSL) modem or a cable modem. Any direct or indirect network connection may be used, including, but not limited to, a telephone modem, an MIB system, an RS232 interface, an auxiliary interface, an optical link, an infrared link, a radio frequency link, a microwave link, a WLAN connection, or another type of wireless connection.

The functional modules 130-133 are devices (e.g., infusion pumps 130-133 of FIG. 1A) for providing care to a patient or for monitoring patient conditions. As shown in FIG. 2, at least one of functional modules 130-133 may be an infusion pump module such as an intravenous infusion pump for delivering medication or other fluid to a patient. For the purposes of this discussion, functional module 130 is an infusion pump module. Each of the functional modules 130-133 may be any patient treatment or monitoring device including, but not limited to, an infusion pump, a syringe pump, a PCA pump, an epidural pump, an enteral pump, a blood pressure monitor, a pulse oximeter, an EKG monitor, an EEG monitor, a heart rate monitor, an intracranial pressure monitor, or the like. Additionally, the functional modules 130-133 may be a printer, a scanner, a bar code reader, a near-field communication reader, an RFID reader, or any other peripheral input, output or input/output device.

Each of the functional modules 130-133 communicates directly or indirectly with the control unit 104, providing overall monitoring and control of the patient care device 102. Additionally, the functional modules 130-133 may be connected physically and electronically in serial fashion to one or both ends of the control unit 104, as shown in FIG. 2.

However, it is recognized that there are other means for connecting the functional modules 130-133 with the control unit 104 that may be utilized without departing from the subject technology. It is also appreciated that devices such as pumps or patient monitoring devices that provide sufficient programmability and connectivity may be capable of operating as stand-alone devices and may communicate directly with the internal healthcare network 236 without being connected through the control unit 104 or a separate interface unit. As described above, additional medical devices or peripheral devices may be connected to the patient care device 102 through one or more auxiliary interfaces 226.

Each of the functional modules 130-133 may include a microprocessor 216, a volatile memory 214, a nonvolatile memory 212, and module-specific components 210. It should be noted that while four functional modules are shown in FIG. 2, any number of devices may be connected directly or indirectly to the control unit 104. The number and type of functional modules described herein are intended to be illustrative, and they in no way limit the scope of the subject technology. The module-specific components 210 include any components necessary for operation of a particular module, such as a pumping mechanism for the functional module 130.

While each of the functional modules 130-133 may be capable of a least some level of independent operation, the control unit 104 monitors and controls overall operation of the patient care device 102. For example, as will be described in more detail below, the control unit 104 provides programming instructions to the functional modules 130-133 and monitors the status of each of the functional modules 130-133.

Medical devices incorporating aspects of the subject technology may be equipped with a network interface module (NIM), allowing the medical device to participate as a node in a network. While for purposes of clarity the subject technology will be described as operating in an Ethernet network environment using the Internet Protocol (IP), it is understood that concepts of the subject technology are equally applicable in other network environments, and such environments are intended to be within the scope of the subject technology.

Data to and from the various data sources can be converted into network-compatible data with existing technology, and movement of the information between the medical device and network can be accomplished by a variety of means. For example, the patient care device 102 and the internal healthcare network 236 may communicate via automated interaction, manual interaction, or a combination of both automated and manual interaction. Automated interaction may be continuous or intermittent and may occur through direct network connection 220, as shown in FIG. 2, or through RS232 links, MIB systems, RF links such as BLUETOOTH, IR links, WLAN, digital cable systems, telephone modems, or other wired or wireless communication means.

Manual interaction between the patient care device 102 and the internal healthcare network 236 involves physically transferring, intermittently or periodically, data between systems using, for example, the user interface device 230, the coded data input device 232, bar codes, computer disks, portable data assistants, memory cards, or any other media for storing data. The communication means in various aspects is bidirectional with access to data from as many points of the distributed data sources as possible. Decision-making can occur at a variety of places within the internal healthcare network 236. For example, and not by way of limitation, decisions can be made in the information system server 242, decision support, a remote data server, hospital department or unit stations, or within the patient care device 102 itself.

With further reference to FIG. 2, the patient care device 102 is capable of operating in several different modes, or personalities, with each personality defined by a configuration database. The configuration database may be a database 226 internal to the patient care device 102, or an external database 244. A particular configuration database is selected based, at least in part, by patient-specific information such as patient location, age, physical characteristics, or medical characteristics.

Medical characteristics include, but are not limited to, patient diagnosis, treatment prescription, medical history, medical records, patient care provider identification, physiological characteristics or psychological characteristics. As used herein, patient-specific information also includes care provider information (e.g., physician identification) or the location of the patient care device 102 location in the hospital or hospital computer network. Patient care information may be entered through any of the network connection 220, the user interface device 230, the data input device 232, or the auxiliary interface 226. Additionally, patient care information may originate from anywhere in the internal healthcare network 236, such as from a pharmacy server, an admissions server, a laboratory, and the like.

The memory of the control unit 104 (e.g., RAM 222 or the disk 228) may contain a drug library, an event log, and/or infusion pump configuration settings, such as profiles to be used in particular practice areas (e.g., ICU, PED, etc.). The control unit 104 memory may be electronically loadable memory such as non-volatile memory (e.g., EEPROM). Drug libraries stored on pumps, which illustratively contain such information as the drug names, ranges of delivery parameter values such as proper concentrations, dosage units, and dose limits, can be used to perform drug-calculation-based infusions in a clinical setting.

A drug library stored within the pump's memory may include clinical order settings such as limits set by the clinical institution for each drug of the library (also termed as "guardrails" herein). Such limits may take the form of maximum and minimum dosages for each drug which may be made dependent on patient factors or other factors associated with delivery of the drug. For example, the dosage limits may vary depending on the weight of the patient or body surface area ("BSA"), depending on the unit or ward of the medical institution in which the drug is being used (for example neonatal care unit (NCU), the intensive care unit (ICU), etc.), and/or other factors.

An alarm may be provided if the nurse sets the pump to operate outside the range between the limits for a particular drug. In some cases, the alarm may be overridden and in other cases it may not. The medical facility may establish "soft" limits for each drug, which may be overridden by the nurse, and "hard" limits which may not. In either case where a limit is exceeded, a pump data log or other processor in communication with the infusion pump may record each such limit event for later analysis where the attempted setting is higher than the maximum or lower than the minimum dosage.

The pump also includes a display for displaying a user interface, including a control panel through which the user can program the programmable controller and a display screen for displaying drug entries from the drug library. Each of the associated sets of drug delivery parameters includes information selected from a group of parameters including drug concentration, drug delivery rate, drug dose, and bolus size. The electronically loaded drug library contains a list of available mode options specifying the units available for expressing drug delivery information, and the drug infusion pump offers the user the list of available mode options from which to make a selection when the electronically loaded drug library is in the pump.

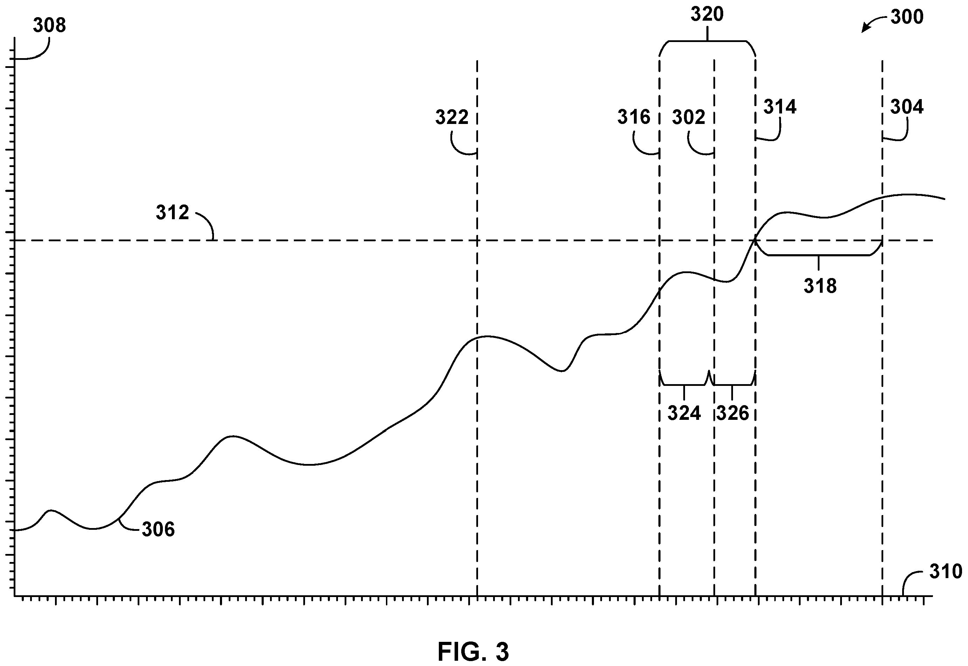

FIG. 3 depicts example clinician response times 302 and 304 to an infusion alarm, according to various aspects of the subject technology. As illustrated, an example line plot 300 includes an infusion metric line 306 that corresponds to the magnitude of an infusion metric (see y-axis 308) over time (see x-axis 310). The line plot 300 also includes a safety threshold line 312 that corresponds to a safety threshold for the infusion metric. The infusion metric line 306 crosses the safety threshold line 312 at time 314, which corresponds to the triggering of an infusion alarm (e.g., an end-of-infusion alarm, an occlusion alarm, an air-in-line alarm, or an end-of-travel alarm).

As discussed in more detail below, in the absence of a pre-alarm notification, a clinician may not respond to the infusion alarm until well after the infusion alarm is triggered (e.g., at time 304). However, if the clinician is informed of the upcoming alarm in advance (e.g., at time 316), the clinician can then respond to the alarm before it triggers (e.g., at time 302). In this manner, providing a pre-alarm notification to the clinician may reduce alarm fatigue, avoid disturbing the patient, and preserve resources (e.g., power, processing time, network traffic, network interface, etc.) of the infusion device.

It should be noted, the infusion metric can be any infusion metric associated with a patient care device 102. For example, the infusion metric can be a runtime of an infusion therapy being performed by an infusion pump 130, a pressure (e.g., a downstream pressure, an upstream pressure) associated with an administrative set 120, an amount of air (e.g., a volume of air, a number of bubbles) in the administrative set 120, or an amount of fluid remaining in a fluid supply 110.

Relatedly, the safety threshold line 312 of the example line plot 300 represents a boundary for the infusion metric line 306. For many infusion therapies, safety requires that certain infusion metrics remain within particular boundaries. An infusion metric passing a boundary may indicate a potential safety hazard. Accordingly, in some implementations, the patient care device 102 is configured to trigger an alarm upon detecting that an infusion metric is not within a safety boundary (e.g., at time 314).

For example, the patient care device 102 may be configured to trigger an end-of-infusion alarm after detecting that a runtime of an infusion therapy exceeds a programmed runtime. As another example, the patient care device 102 may be configured to trigger an occlusion alarm after detecting that a pressure in a downstream portion of an administrative set 120-123 is greater than a certain pressure. As yet another example, the patient care device 102 may be configured to trigger an air-in-line alarm after detecting that an amount of air in an administrative set 120-123 is greater than a certain amount of air. As a further example, the patient care device 102 may be configured to trigger an end-of-travel alarm after detecting that a volume remaining in a fluid supply 110-113 is less than a certain volume.

Depending on the clinician's ability to respond to the alarm (e.g., based on availability or proximity), the alarm may sound for a minute or more (e.g., during time period 318) before the clinician is able to respond to the alarm and resolve it. While the alarm is sounding, the patient receiving care from the patient care device 102 may be awoken, startled, or otherwise irritated. This is especially true for the high-volume alarms required by certain countries' regulations. For example, in the United States, the Food and Drug Administration requires a high-priority alarm for indicating end of infusion.

Accordingly, the patient care device 102 or a device connected thereto (e.g., the device terminal 240 of FIG. 2) may generate and provide a pre-alarm notification (e.g., to a clinician or other associated device) after determining (e.g., at time 316) that the alarm will or is likely to trigger. Ideally, the clinician or a clinical control message will arrive at the patient care device 102 before (e.g., at time 312) the alarm triggers, thus preventing the alarm from triggering or at least minimizing the amount of time for which it sounds. In this manner, sending pre-alarm notifications to the clinician or other device configured to provide clinical control messages may improve resource utilization at the infusion device, enhance the overall patient experience, decrease clinician alarm fatigue, and preserve resources (e.g., power, processing time, network traffic, network interface, etc.) of the infusion device.

Additionally, the patient care device 102 may be configured to determine a lead time threshold. For example, the lead time threshold may be a window of time (e.g., time period 320) prior to a predicted alarm time. The lead time threshold may thus be used in determining whether or when to send the pre-alarm notification or take other prophylactic actions to avert or delay the predicted alarm.

In some implementations, the patient care device 102 is configured to send the notification responsive to the current time corresponding to (e.g., being within) the lead time threshold. For example, if the patient care device 102 predicts at time 316 an upcoming infusion alarm and the lead time threshold is time period 320, then the patient care device 102 may send the notification immediately after predicting the upcoming alarm because the current time (e.g., time 316) is within the lead time threshold (e.g., time period 320).

On the other hand, in some implementations, the patient care device 102 is configured to not send the notification responsive to the current time not being within the lead time threshold. For example, if the patient care device 102 predicts at time 322 an upcoming infusion alarm and the lead time threshold is time period 320, then the patient care device 102 may not send the notification because the current time (e.g., time 322) is not within the lead time threshold (e.g., time period 320). Rather, the patient care device 102 may wait until the start of the lead time threshold (e.g., at time 316) to send the notification.

Further, in some implementations, the lead time threshold does not include or abut the predicted alarm time (see time period 324). In this manner, the gap between the lead time threshold and the predicted alarm time (see time period 326) may account for instances where sending a pre-alarm notification would be ineffective because the clinician would not have enough time to respond to the notification after receiving it and before the alarm is predicted to trigger. Accordingly, in some of these implementations, the patient care device 102 is likewise configured not to send the notification responsive to the current time not being within the lead time threshold.

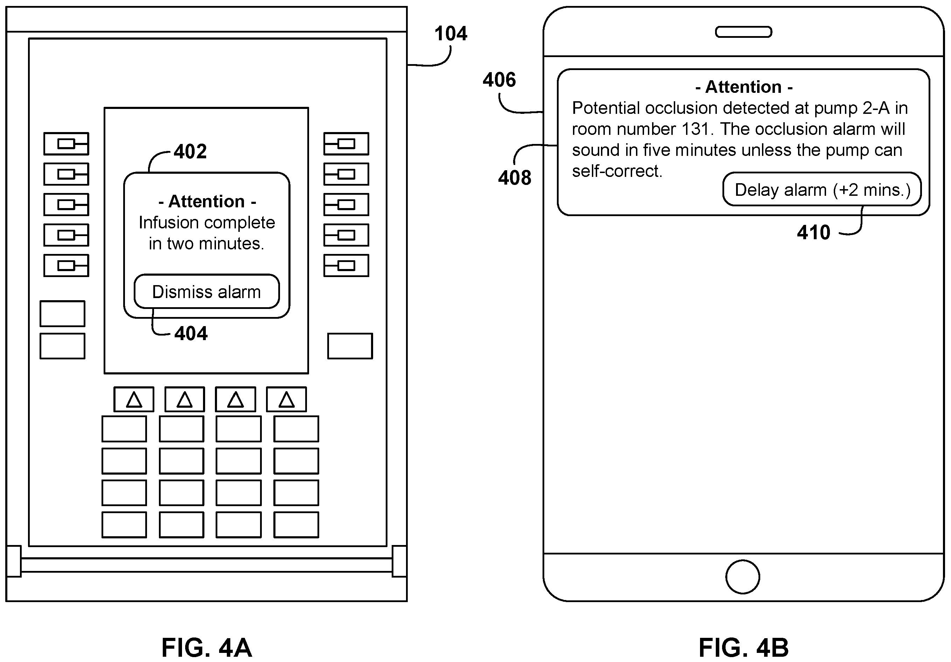

FIGS. 4A and 4B depict example pre-alarm notifications 402 and 408 displayed at a control unit 104 and a mobile device 406, respectively, according to various aspects of the subject technology. The pre-alarm notifications 402 and 408 each contain information regarding an upcoming alarm at a patient care device 102, including the type of the alarm and when it is expected to trigger. The pre-alarm notifications 402 and 408 also include buttons 404 and 410 for dismissing, delaying, or otherwise altering the alarm. While specific examples for dismissing, delaying, or otherwise altering the alarm are described herein, it is understood that any alarm monitored by the subject technology may be subject to early detection and dismissed, delayed, or otherwise altered.

The first pre-alarm notification 402 regards an end-of-infusion alarm. The notification 402 indicates that an infusion therapy being administered by the control unit 104 (e.g., via an attached infusion pump 130-133) will complete in two minutes. Additionally, the notification includes a button 404 that allows a user to dismiss the alarm before it triggers. For example, selecting the button 404 may cause the control unit 104 or a functional module 130-133 attached thereto to forego triggering the end-of-infusion alarm.

The second pre-alarm notification 408 regards a potential occlusion alarm. Unlike the end-of-infusion alarm of the first notification 402, the occlusion alarm is not guaranteed to trigger. Rather, the second notification 408 indicates that a potential occlusion was detected and the occlusion alarm will trigger unless the occlusion can be corrected (e.g., by restarting the infusion pump 130-133 or by adjusting a flow rate of the infusion pump 130-133). The second notification 408 also includes a button 410 for delaying the alarm by two minutes. In some implementations, the notification 408 may not include an option for the user to delay the alarm if the alarm condition is of a particular severity.

In some implementations, a pre-alarm notification is displayed at the control unit 104 after a clinician logs into the control unit 104. For example, the control unit 104 may display a notification indicating each of the upcoming or predicted alarms associated with functional modules 130-133 connected to the control unit 104. Similarly, in some implementations, a pre-alarm notification is displayed at the mobile device 406 after a clinician logs into the mobile device 406 or uses the mobile device to connect to the internal healthcare network 236. The pre-alarm notification may indicate each of the upcoming or predicted alarms associated with patients for which the clinician is responsible.

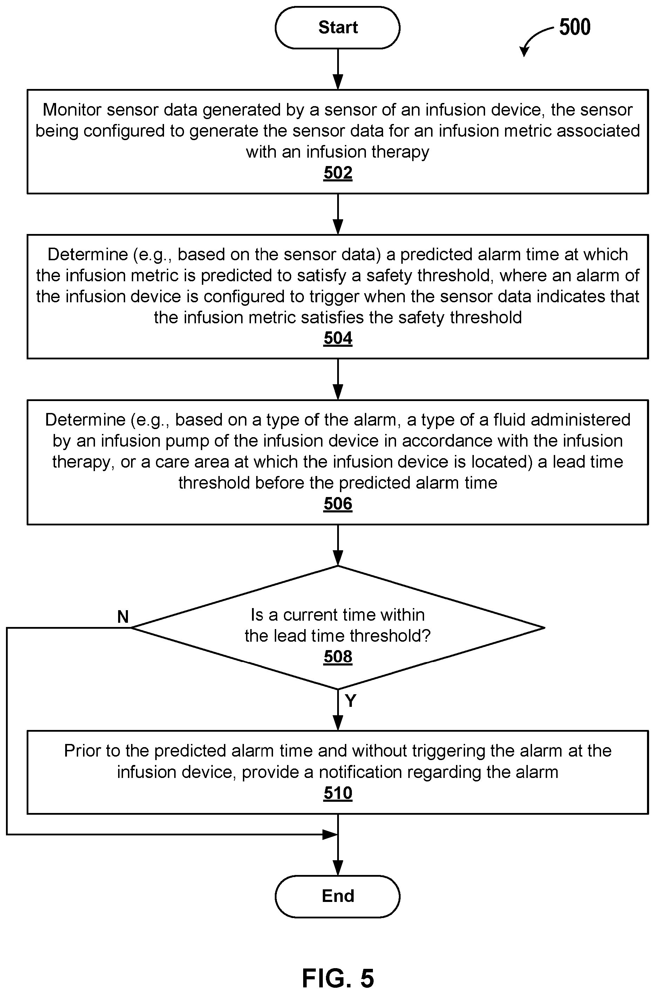

FIG. 5 depicts an example process 500 for managing alarms of an infusion device, according to aspects of the subject technology. One or more blocks of the process 500 may be implemented, for example, by one or more computing devices, such as a patient care device 102, a control unit 104, a device terminal 240, or a mobile device 406.

In some implementations, one or more of the blocks may be implemented based on one or more machine learning algorithms. In some implementations, one or more of the blocks may be implemented apart from other blocks, and by one or more different processors or devices. Further, for explanatory purposes, the blocks of the process 500 are described as occurring in serial, or linearly. However, multiple blocks of the process 500 may occur in parallel. Additionally, the blocks of the process 500 need not be performed in the order shown and one or more of the blocks of the process 500 need not be performed.

In the depicted example, a processor (e.g., CPU 218 of FIG. 2) of an infusion device (e.g., patient care device 102 of FIGS. 1A-2) monitors sensor data generated by a sensor of the infusion device, the sensor being configured to generate sensor data for an infusion metric associated with an infusion therapy (502). The sensor may be, for example, an end-of-infusion detector, an occlusion detector, an air-in-line detector, or an end-of-travel sensor. For illustrative purposes and without limiting the disclosure, the infusion device is referred to herein as patient care device 102.

In addition to monitoring the sensor data, the processor also determines (e.g., based on the sensor data) a predicted alarm time at which the infusion metric is predicted to satisfy a safety threshold (504), where an alarm of the patient care device 102 is configured to trigger when the sensor data indicates that the infusion metric corresponds to the safety threshold. The alarm of the patient care device 102 may be associated with a respective infusion metric and safety threshold. In some implementations, the alarm may be associated with multiple safety thresholds, for instance, setting upper and lower bounds for the infusion metric.

As an example, the safety threshold may be a programmed runtime of an infusion and the infusion metric the actual runtime of the infusion. The patient care device 102 may include an end-of-infusion alarm configured to trigger when an infusion runtime exceeds the programmed runtime. As another example, the patient care device 102 may include an occlusion detector configured to trigger an occlusion alarm when a pressure in a downstream portion of an administrative set 120 (infusion metric) exceeds a programmed amount of pressure (safety threshold). As yet another example, the patient care device 102 may include an air-in-line detector configured to trigger an air-in-line alarm when an amount of air detected in the administrative set 120 (infusion metric) exceeds a programmed amount of air (safety threshold). And, as a further example, the patient care device 102 may include an end-of-travel sensor configured to trigger an end-of-travel alarm when a volume remaining in a fluid supply 110 (infusion metric) is less than a programmed minimum volume (safety threshold).

In some implementations, the processor determines a predicted alarm time independently of monitored sensor data. For a given alarm, for example, the processor may monitor internal infusion data that is independent of sensor data and compare the infusion data to a respective safety threshold. For example, the processor may compare an infusion runtime against a programmed runtime. As another example, the processor may compare an estimated infused volume (e.g., based on infusion runtime and programmed flow rate) against a programmed volume-to-be-infused. Internal infusion data can be used, in addition to or in lieu of sensor data, in predicting the time of an upcoming alarm without departing from the scope of the present disclosure.

Furthermore, the processor also determines a lead time threshold before the predicted alarm time (506). As used herein, "lead time threshold" describes a window of time for notifying a clinician of the upcoming alarm. In some instances, the lead time threshold is the window of time immediately before and leading up to the predicted alarm time. For example, if an alarm is predicted to trigger at 5:30 PM, the lead time threshold may be a ten-minute window between 5:20 PM and 5:30 PM. However, in other instances, the lead time threshold does not abut the predicted alarm time. Given a 5:30 PM predicted alarm time, for example, the lead time threshold may include a five-minute window between 5:23 PM and 5:28 PM. When the current time is within the window of time defined by the lead time threshold, the current time is said to be within the lead time threshold.

In addition to the predicted alarm time, the lead time threshold is based on a type of the alarm, a type of fluid administered by an infusion pump 130 of the patient care device 102 in accordance with the infusion therapy, or a care area at which the patient care device 102 is located. For example, the lead time threshold may scale to account for the amount of time required to handle the type of the alarm. Additionally, the lead time threshold may scale to account for a severity of a safety hazard indicated by the type of the alarm. Further, the lead time threshold may scale to account for whether the fluid is a saline solution, nutrition, or a medication. Also, the lead time threshold may scale to account for whether the care area indicates a heightened need for alarm responsiveness, such as in an intensive care unit.

After determining the predicted alarm time (504) and the lead time threshold (506), the processor determines whether the current time is within the lead time threshold (508). If the processor determines that the current time is not within the lead time threshold (508-N), the processor may then complete the process 500 (e.g., forgoing sending the notification). For example, this includes instances where the current time is earlier than the lead time threshold (compare time 322 and time period 320 of FIG. 3), such that it may not yet be worth notifying the clinician of the upcoming alarm. In some implementations, the processor may wait until the current time is within the lead time threshold to provide the notification. The current time may also not be within the lead time threshold where the current time is later than the lead time threshold (compare time 302, and time period 324 of FIG. 3), such that the alarm is predicted to trigger soon enough that notifying the clinician in advance would not result in a quicker response time.

On the other hand, if the processor determines that the current time is within the lead time threshold (508-Y), the processor then provides a notification regarding the alarm (510) prior to the predicted alarm time and without triggering the alarm at the infusion device. For example, providing the notification may include providing the notification for display via a display 114 of the patient care device 102. As another example, providing the notification may include providing the notification for display via a device connected to the patient care device (e.g., device terminal 240 of FIG. 2), such as a mobile device or a clinician computer.

In some implementations, the lead time threshold is retrieved from a lookup table stored on an internal disk 228, an information system server 242, or an external database 244. The lookup table may be indexed, for example, using the type of the alarm, the type of the fluid, or the care area at which the patient care device 102 is located.

Determining the lead time threshold (506) is further based on collecting historical data for clinician response times to notifications. The historical data is provided to a machine-learning model, and a new lead time threshold is received from the machine-learning model responsive to providing the historical data. In some implementation, an initial lead time threshold may be updated or revised based on the new lead time threshold. The machine-learning model may accept as input values sensor data, an infusion type, a drug type, a care area, an alarm presentation status, an alarm response time, a clinician identifier, or other recorded historical infusion information. Based on the input values, the machine-learning model may provide a recommended lead time threshold for the input values. In some implementations, the recommendation may be based on a regression, mean, average, or other analytical processing of the historical data.

Revising the initial lead time threshold may include comparing the initial lead time threshold to the new lead time threshold and then changing the initial lead time threshold to a revised lead time threshold if the new lead time threshold is substantially different (e.g., 10% or 25% different) from the initial lead time threshold. This process can then be repeated by collecting additional historical data, providing the additional historical data to the machine-learning model, receiving another new lead time threshold, and revising the revised lead time threshold based on the other new lead time threshold. In this manner the machine-learning model can be used to continuously refine the lead time threshold for a given type of the alarm, type of the fluid, care area, or a combination thereof.

In some implementations, the processor is further configured to respond to the clinician interacting with the notification. For example, the clinician may interact with the notification by selecting it (see, e.g., buttons 404 and 410 of FIG. 4) or dismissing it. The processor may be configured, for example, adjust the safety threshold responsive to the clinician interacting with the notification such that the alarm will not trigger prior to or at the predicted alarm time. Additionally, or alternatively, the processor may be configured to disable the alarm responsive to the clinician interacting with the notification such that the alarm will not trigger when the sensor data indicates that the infusion metric satisfies the safety threshold. In some implementations, the processor is configured to adjust the safety threshold or disable the alarm for a predetermined amount of time (e.g., based on the type of the alarm, the type of the fluid, or the care area at which the infusion device is located).

In some implementations, the processor is further configured to respond to the clinician logging into the patient care device 102. For example, after the clinician logs into the patient care device 102, the processor may inform the clinician of an upcoming alarm and a predicted alarm time for the alarm. Additionally, the processor may prompt the clinician to indicate whether to disable the alarm and, responsive to receiving an indication from the clinician to disable the alarm, disable the alarm for a predetermined amount of time (e.g., based on the type of the alarm, the type of the fluid, or the care area at which the infusion device is located).

In some implementations, the patient care device 102 includes a light indicator, also referred to as a "lighthouse." Accordingly, the processor may set a brightness or a color of the light indicator based on a difference between the infusion metric and the safety threshold. For example, the processor may increase the brightness of the light indicator as the infusion metric approaches or passes the safety threshold. As another example, the processor may set the color of the light indicator to green when the difference between the infusion metric and the safety threshold indicates that the infusion metric does not satisfy (e.g., exceed) the safety threshold. Or the processor may set the color of the light indicator to red when the difference between the infusion metric and the safety threshold indicates that the infusion metric does satisfy (e.g., exceed) the safety threshold.

According to various implementations, the lead time threshold sets an ideal notification time (e.g., the predicted alarm time minus the duration of the lead time threshold). The processor may determine at run time whether the notification should be sent at the beginning of the lead time threshold or at another time (e.g., within the lead time threshold). If the notification is sent too early, the clinician may feel that they have plenty of time and procrastinate responding to the upcoming alarm. However, if the notification is sent too late, the clinician may not have enough time to respond to the upcoming alarm. Of course, clinician response behavior may be unique to each clinician.

Accordingly, in some implementations, the system may forego sending the alarm when a variance between the estimated response time and the determined lead time satisfies a given threshold variance. An estimated response time may be determined based on historical data corresponding to response times for the clinician, or for clinicians in the particular care area. The estimated response time may then be considered in determining whether a clinician responding to the instant notification would otherwise avoid the upcoming alarm (e.g., in response to a pre-alarm notification).

For example, if the predicted alarm time is 5:30 PM, the lead time threshold is 5:20 PM to 5:30 PM, but the estimated response time is 15 minutes, then the system may determine that sending the notification would not avoid the alarm and forego the notification (thus allowing the alarm to be triggered normally). The threshold variance is this example may be 5 minutes or 50% of the duration of the lead time threshold. In some implementations, the estimated response time and/or the threshold variance may be determined based on the type of the alarm, the type of the fluid, and/or the care area at which the infusion device is located.

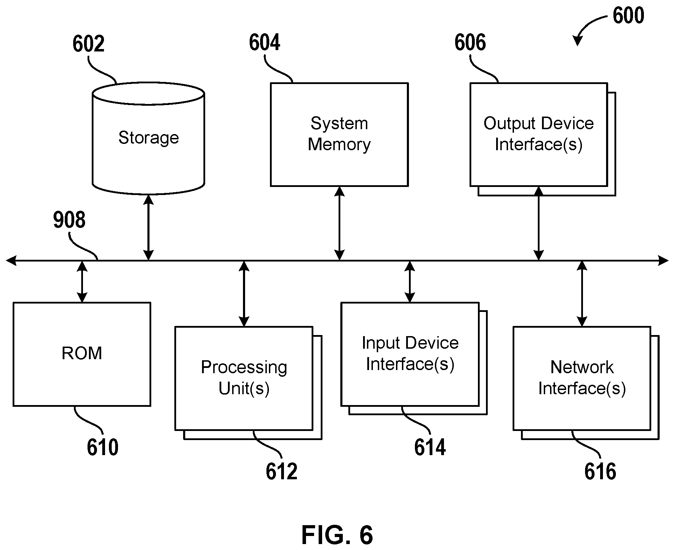

FIG. 6 is a conceptual diagram illustrating an example electronic system 600 for managing alarms of an infusion device, according to aspects of the subject technology. Electronic system 600 may be implemented by a computing device for execution of software associated with portions or steps of process 500, or components and methods provided by FIGS. 1-4. In this regard, electronic system 600 may include a patient care device 102, a device terminal 240, or a mobile device 406. The electronic system 600 may also include a specifically-configured personal computer or a mobile device for infusion such as a smartphone, tablet computer, laptop, PDA, an augmented reality device, a wearable such as a watch or band or glasses, or combination thereof, or other touch screen or television with one or more processors embedded therein or coupled thereto, or any other sort of computer-related electronic device having network connectivity.

The electronic system 600 may also include various types of computer readable media and interfaces for various other types of computer readable media. In the depicted example, electronic system 600 includes a bus 608, a processing unit(s) 612, a system memory 604, a read-only memory (ROM) 610, a permanent storage device 602, an input device interface(s) 614, an output device interface(s) 606, and a network interface(s) 616. In some implementations, electronic system 600 may include or be integrated with other computing devices or circuitry for operation of the various components and methods previously described.

The bus 608 collectively represents all system, peripheral, and chipset buses that communicatively connect the numerous internal devices of electronic system 600. For instance, bus 608 communicatively connects processing unit(s) 612 with ROM 610, the system memory 604, and permanent storage device 602.

From these various memory units, processing unit(s) 612 retrieves instructions to execute and data to process in order to execute the processes of the subject disclosure. Processing unit(s) 612 can be a single processor or a multi-core processor in different implementations.

The ROM 610 stores static data and instructions that are needed by processing unit(s) 612 and other modules of the electronic system. Permanent storage device 602, on the other hand, is a read-and-write memory device. This device is a non-volatile memory unit that stores instructions and data even when electronic system 600 is powered off. Some implementations of the subject disclosure use a mass-storage device (such as a magnetic or optical disk and its corresponding disk drive) as permanent storage device 602. Other implementations use a removable storage device (such as a floppy disk, flash drive, and its corresponding disk drive) as permanent storage device 602.

Like permanent storage device 602, the system memory 604 is a read-and-write memory device. However, unlike the permanent storage device 602, the system memory 604 is a volatile read-and-write memory, such as random-access memory (RAM). System memory 604 stores some of the instructions and data that the processor needs at runtime. In some implementations, the processes of the subject disclosure are stored in system memory 604, permanent storage device 602, and/or ROM 610. From these various memory units, processing unit(s) 612 retrieves instructions to execute and data to process, in order to execute the processes of some implementations.

Bus 608 also connects to input device interface(s) 614 and output device interface(s) 606. Input device interface(s) 614 enables the user to communicate information and select commands to the electronic system. Input devices used with input device interface(s) 614 include, for example, alphanumeric keyboards and pointing devices (also called "cursor control devices"). Output device interface(s) 606 enables, for example, the display of images generated by electronic system 600. Output devices used with output device interface(s) 606 include, for example, printers and display devices, such as cathode ray tubes (CRT) or liquid crystal displays (LCD). Some implementations include devices (e.g., touchscreens) that function as both input and output devices.

Furthermore, bus 608 also couples electronic system 600 to a network (not shown) through network interface(s) 616. Network interface(s) 616 may include, for example, a wireless access point (e.g., Bluetooth or Wi-Fi) or radio circuitry for connecting to a wireless access point. Network interface(s) 616 may also include hardware (e.g., ethernet hardware) for connecting the computer to a part of a network of computers such as a local area network (LAN), a wide area network (WAN), wireless LAN, an intranet, or a network of networks, such as the Internet. Any or all components of electronic system 600 can be used in conjunction with the subject disclosure when specifically configured with one of more of the features described.

These functions described above can be implemented in computer software, firmware, or hardware. The techniques can be implemented using one or more computer program products. Programmable processors and computers can be included in or packaged as mobile devices. The processes and logic flows can be performed by one or more programmable processors and by programmable logic circuitry. General and special purpose computing devices and storage devices can be interconnected through communication networks.

Some implementations include electronic components, such as microprocessors, storage and memory that store computer program instructions in a machine-readable or computer-readable medium (also referred to as computer-readable storage media, machine-readable media, or machine-readable storage media). Some examples of such computer-readable media include RAM, ROM, read-only compact discs (CD-ROM), recordable compact discs (CD-R), rewritable compact discs (CD-RW), read-only digital versatile discs (e.g., DVD-ROM, dual-layer DVD-ROM), a variety of recordable/rewritable DVDs (e.g., DVD-RAM, DVD-RW, DVD+RW, etc.), flash memory (e.g., SD cards, mini-SD cards, micro-SD cards, etc.), magnetic and/or solid state hard drives, read-only and recordable Blu-Ray^{®} discs, ultra density optical discs, other optical or magnetic media, and floppy disks. The computer-readable media can store a computer program that is executable by at least one processing unit and includes sets of instructions for performing various operations. Examples of computer programs or computer code include machine code, such as is produced by a compiler, and files including higher-level code that are executed by a computer, an electronic component, or a microprocessor using an interpreter.

While the above discussion primarily refers to microprocessor or multi-core processors that execute software, some implementations are performed by one or more integrated circuits, such as application specific integrated circuits (ASICs) or field-programmable gate arrays (FPGAs). In some implementations, such integrated circuits execute instructions that are stored on the circuit itself.

As used in this specification and any claims of this application, the terms "computer," "server," "processor," and "memory" all refer to electronic or other technological devices specifically configured with one or more of the features described above. These terms exclude people or groups of people. For the purposes of the specification, the terms display or displaying means displaying on an electronic device. As used in this specification and any claims of this application, the terms "computer readable medium" and "computer readable media" are entirely restricted to tangible, physical objects that store information in a form that is readable by a computer. These terms exclude any wireless signals, wired download signals, and any other ephemeral signals.

To provide for interaction with a user, implementations of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well. For example, feedback provided to the user can be any form of sensory feedback (e.g., visual feedback, auditory feedback, tactile feedback), and input from the user can be received in forms such as acoustic, speech, gesture, or tactile input. In addition, a computer can interact with a user by sending documents to and receiving documents from a device that is used by the user (e.g., by sending web pages to a web browser on a user's client device in response to requests received from the web browser).

Implementations of the subject matter described in this specification can be implemented in a specifically configured computing system that includes a back end component (e.g., a data server), or that includes a specifically configured middleware component (e.g., an application server), or that includes a specifically configured front end component (e.g., a client computer having a graphical user interface or a Web browser through which a user can interact with an implementation of the subject matter described in this specification), or any combination of one or more such back end, middleware, or front end components. The components of the system can be interconnected by one or more forms or mediums of digital data communication, such as a communication network. Examples of communication networks include a LAN and a WAN, an inter-network (e.g., the Internet), and peer-to-peer networks (e.g., ad hoc peer-to-peer networks).

The computing system can include specifically configured clients and servers. A client and server are generally remote from each other and may interact through a communication network. The relationship of client and server arises by virtue of computer programs running on the respective computers and having a client-server relationship to each other. In some implementations, a server transmits data (e.g., an HTML page) to a client device (e.g., for purposes of displaying data to and receiving user input from a user interacting with the client device). Data generated at the client device (e.g., a result of the user interaction) can be received from the client device at the server.

Those of skill in the art will appreciate that the various illustrative blocks, modules, elements, components, methods, and algorithms described herein may be implemented as electronic hardware, computer software, or a combination thereof. To illustrate this interchangeability of hardware and software, various illustrative blocks, modules, elements, components, methods, and algorithms have been described above generally in terms of their functionality. Whether such functionality is implemented as hardware or software depends upon the particular application and design constraints imposed on the overall system. The described functionality may be implemented in varying ways for each particular application. Various components and blocks may be arranged differently (e.g., arranged in a different order, or partitioned in a different way.

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order and are not meant to be limited to the specific order or hierarchy presented.

Illustration of Subject Technology as Clauses:
Various examples of aspects of the disclosure are described as numbered clauses (1, 2, 3, etc.) for convenience. These are provided as examples, and do not limit the subject technology. Identifications of the figures and reference numbers are provided below merely as examples and for illustrative purposes, and the clauses are not limited by those identifications.

Clause 1. An infusion device, comprising: an infusion pump configured to deliver a fluid in accordance with an infusion therapy; a sensor configured to generate sensor data for an infusion metric associated with the infusion therapy; an alarm configured to trigger when the sensor data indicates that the infusion metric satisfies a safety threshold; and a processor configured to: monitor the sensor data generated by the sensor; determine, based on the sensor data, a predicted alarm time at which the infusion metric is predicted to satisfy the safety threshold; determine, based on (i) a type of the alarm, (ii) a type of the fluid, or (iii) a care area at which the infusion device is located, a lead time threshold before the predicted alarm time; and based on a current time being within the lead time threshold, and prior to the predicted alarm time and without triggering the alarm at the infusion device, provide a notification regarding the alarm.

Clause 2. The infusion device of Clause 1, wherein determining the lead time threshold is further based on (i) collecting historical data for clinician response times to notifications provided using an initial lead time threshold, (ii) providing the historical data to a machine-learning model, (iii) receiving a new lead time threshold from the machine-learning model responsive to providing the historical data, and (iv) revising the initial lead time threshold based on the new lead time threshold.

Clause 3. The infusion device of Clause 2, wherein the initial lead time threshold is retrieved from a lookup table using the type of the alarm, the type of the fluid, or the care area at which the infusion device is located.

Clause 4. The infusion device of any one of Clauses 1 through 3, wherein the processor is further configured to, responsive to a user interacting with the notification: adjust the safety threshold for a predetermined amount of time, such that the alarm will not trigger prior to or at the predicted alarm time; or disable the alarm for the predetermined amount of time, such that the alarm will not trigger when the sensor data indicates that the infusion metric satisfies the safety threshold.

Clause 5. The infusion device of any one of Clauses 1 through 4, wherein the processor is further configured to, responsive to a user logging into the infusion device and after determining the predicted alarm time: provide the notification for display via a display device of the infusion device; prompt the user to indicate whether to disable the alarm; and responsive to receiving an indication from the user to disable the alarm, disable the alarm for a predetermined amount of time.

Clause 6. The infusion device of Clause 4 or 5, wherein the predetermined amount of time is based on the type of the alarm, the type of the fluid, or the care area at which the infusion device is located.

Clause 7. The infusion device of any one of Clauses 1 through 6, wherein the infusion device further comprises a light indicator, and the processor is further configured to adjust a brightness or a color of the light indicator based on a difference between the infusion metric and the safety threshold.

Clause 8. The infusion device of any one of Clauses 1 through 7, wherein the processor is further configured to: determine an estimated response time for the alarm based on historical response times for the type of the alarm and the care area at which the infusion device is located; and provide the notification only when the response time is within the lead time threshold, otherwise, forgo providing the notification.

Clause 9. The infusion device of any one of Clauses 1 through 8, wherein providing the notification comprises: identifying a clinician associated with the infusion device; and sending the notification to a mobile device associated with the clinician.

Clause 10. The infusion device of any one of Clauses 1 through 9, wherein the alarm comprises an air-in-line alarm, an occlusion alarm, an end-of-travel alarm, or an end-of-infusion alarm.

Clause 11. A computer-implemented method for managing alarms for an infusion device, the computer-implemented method comprising: monitoring sensor data generated by a sensor of an infusion device, the sensor being configured to generate the sensor data for an infusion metric associated with an infusion therapy; determining, based on the sensor data, a predicted alarm time at which the infusion metric is predicted to satisfy a safety threshold, wherein an alarm of the infusion device is configured to trigger when the sensor data indicates that the infusion metric satisfies the safety threshold; determining, based on (i) a type of the alarm, (ii) a type of a fluid administered by an infusion pump of the infusion device in accordance with the infusion therapy, or (iii) a care area at which the infusion device is located, a lead time threshold before the predicted alarm time; and based on a current time being within the lead time threshold, and prior to the predicted alarm time and without triggering the alarm at the infusion device, providing a notification regarding the alarm.

Clause 12. The computer-implemented method of Clause 11, further comprising, responsive to a user interacting with the notification: adjusting the safety threshold for a predetermined amount of time, such that the alarm will not trigger prior to or at the predicted alarm time; or disabling the alarm for the predetermined amount of time, such that the alarm will not trigger when the sensor data indicates that the infusion metric satisfies the safety threshold.

Clause 13. The computer-implemented method of Clause 11 or 12, further comprising, responsive to a user logging into the infusion device and after determining the predicted alarm time: providing the notification for display via a display device of the infusion device; prompting the user to indicate whether to disable the alarm; and responsive to receiving an indication from the user to disable the alarm, disabling the alarm for a predetermined amount of time.

Clause 14. The computer-implemented method of Clause 11, further comprising: determining an estimated response time for the alarm based on historical response times for the type of the alarm and the care area at which the infusion device is located; and providing the notification only when the response time is within the lead time threshold, otherwise forgoing providing the notification.

Clause 15. A non-transitory, machine-readable storage medium embodying instructions that, when executed by a machine, facilitate the machine to perform the computer-implemented method of any one of Clauses 11-14.

Further Consideration:
It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

The previous description is provided to enable any person skilled in the art to practice the various aspects described herein. The previous description provides various examples of the subject technology, and the subject technology is not limited to these examples. Various modifications to these aspects will be readily apparent to those skilled in the art, and the generic principles defined herein may be applied to other aspects.

Thus, the claims are not intended to be limited to the aspects shown herein but are to be accorded the full scope consistent with the language of the claims. For example, reference to an element in the singular is not intended to mean "one and only one" unless specifically so stated, but rather "one or more." Moreover, unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention described herein.

The predicate words "configured to," "operable to," and "programmed to" do not imply any particular tangible or intangible modification of a subject, but, rather, are intended to be used interchangeably. For example, a processor configured to monitor and control an operation or a component, may also mean the processor being programmed to monitor and control the operation or the processor being operable to monitor and control the operation. Likewise, a processor configured to execute code can be construed as a processor programmed to execute code or operable to execute code.

The term "automatic," as used herein, may include performance by a computer or machine without user intervention, for example, by instructions responsive to a predicate action by the computer or machine or other initiation mechanism. The word "example" is used herein to mean "serving as an example or illustration." Any aspect or design described herein as "example" is not necessarily to be construed as preferred or advantageous over other aspects or designs.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "implementation" does not imply that such implementation is essential to the subject technology or that such implementation applies to all configurations of the subject technology. A disclosure relating to an implementation may apply to all implementations, or one or more implementations. An implementation may provide one or more examples. A phrase such as an "implementation" may refer to one or more implementations and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such as a "configuration" may refer to one or more configurations and vice versa.

As used herein a "user interface" (also referred to as an interactive user interface, a graphical user interface, or a UI) may refer to a network-based interface including data fields or other control elements for receiving input signals or providing electronic information or for providing information to the user in response to any received input signals. Control elements may include dials, buttons, icons, selectable areas, or other perceivable indicia presented via the UI that, when interacted with (e.g., clicked, touched, selected, etc.), initiates an exchange of data for the device presenting the UI. A UI may be implemented in whole or in part using technologies such as hyper-text mark-up language (HTML), FLASH^{™}, JAVA^{™}, .NET^{™}, C, C++, web services, or rich site summary (RSS). In some implementations, a UI may be included in a stand-alone client (for example, thick client, fat client) configured to communicate (e.g., send or receive data) in accordance with one or more of the aspects described. The communication may be to or from a medical device or server in communication therewith.

As used herein, the terms "determine" or "determining" encompass a wide variety of actions. For example, "determining" may include calculating, computing, processing, deriving, generating, obtaining, looking up (e.g., looking up in a table, a database, or another data structure), ascertaining and the like via a hardware element without user intervention. Also, "determining" may include receiving (e.g., receiving information), accessing (e.g., accessing data in a memory) and the like via a hardware element without user intervention. "Determining" may include resolving, selecting, choosing, establishing, and the like via a hardware element without user intervention.

As used herein, the terms "provide" or "providing" encompass a wide variety of actions. For example, "providing" may include storing a value in a location of a storage device for subsequent retrieval, transmitting a value directly to the recipient via at least one wired or wireless communication medium, transmitting or storing a reference to a value, and the like.

"Providing" may also include encoding, decoding, encrypting, decrypting, validating, verifying, and the like via a hardware element.

As used herein, the term "message" encompasses a wide variety of formats for communicating (e.g., transmitting or receiving) information. A message may include a machine-readable aggregation of information such as an XML document, fixed field message, comma separated message, JSON, a custom mode, or the like. A message may, in some implementations, include a signal utilized to transmit one or more representations of the information. While recited in the singular, it will be understood that a message may be composed, transmitted, stored, received, etc. in multiple parts.

As used herein, the term "selectively" or "selective" may encompass a wide variety of actions. For example, a "selective" process may include determining one option from multiple options. A "selective" process may include one or more of: dynamically determined inputs, preconfigured inputs, or user-initiated inputs for making the determination. In some implementations, an n-input switch may be included to provide selective functionality where n is the number of inputs used to make the selection.

As used herein, the terms "correspond" or "corresponding" encompasses a structural, functional, quantitative and/or qualitative correlation or relationship between two or more objects, data sets, information and/or the like, preferably where the correspondence or relationship may be used to translate one or more of the two or more objects, data sets, information and/or the like so to appear to be the same or equal. Correspondence may be assessed using one or more of a threshold, a value range, fu5y logic, pattern matching, a machine learning assessment model, or combinations thereof.

In some implementations, data generated or detected can be forwarded to a "remote" device or location, where "remote," means a location or device other than the location or device at which the program is executed. For example, a remote location could be another location (e.g., office, lab, etc.) in the same city, another location in a different city, another location in a different state, another location in a different country, etc. As such, when one item is indicated as being "remote" from another, what is meant is that the two items can be in the same room but separated, or at least in different rooms or different buildings, and can be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. Examples of communicating media include radio or infra-red transmission channels as well as a network connection to another computer or networked device, and the internet or including email transmissions and information recorded on websites and the like.

## Claims

1. An infusion device, comprising:
an infusion pump configured to deliver a fluid in accordance with an infusion therapy;
a sensor configured to generate sensor data for an infusion metric associated with the infusion therapy;
an alarm configured to trigger when the sensor data indicates that the infusion metric satisfies a safety threshold; and
a processor configured to:
monitor the sensor data generated by the sensor;
determine, based on the sensor data, a predicted alarm time at which the infusion metric is predicted to satisfy the safety threshold;
determine, before the predicted alarm time, a lead time threshold based on (i) a type of the alarm, (ii) a type of the fluid, or (iii) a care area at which the infusion device is located, and further based on (iv) collecting historical data for clinician response times to notifications provided using an initial lead time threshold, (v) providing the historical data to a machine-learning model, (vi) receiving a new lead time threshold from the machine-learning model responsive to providing the historical data, and (vii) revising the initial lead time threshold based on the new lead time threshold; and
based on a current time being within the lead time threshold, and prior to the predicted alarm time and without triggering the alarm at the infusion device, provide a notification regarding the alarm.

2. The infusion device of Claim 1, wherein the initial lead time threshold is retrieved from a lookup table using the type of the alarm, the type of the fluid, or the care area at which the infusion device is located.

3. The infusion device of Claim 1, wherein the processor is further configured to, responsive to a user interacting with the notification:
adjust the safety threshold for a predetermined amount of time, such that the alarm will not trigger prior to or at the predicted alarm time; or
disable the alarm for the predetermined amount of time, such that the alarm will not trigger when the sensor data indicates that the infusion metric satisfies the safety threshold.

4. The infusion device of Claim 1, wherein the processor is further configured to, responsive to a user logging into the infusion device and after determining the predicted alarm time:
provide the notification for display via a display device of the infusion device;
prompt the user to indicate whether to disable the alarm; and
responsive to receiving an indication from the user to disable the alarm, disable the alarm for a predetermined amount of time.

5. The infusion device of Claim 3 or 4, wherein the predetermined amount of time is based on the type of the alarm, the type of the fluid, or the care area at which the infusion device is located.

6. The infusion device of Claim 1, wherein the infusion device further comprises a light indicator, and the processor is further configured to adjust a brightness or a color of the light indicator based on a difference between the infusion metric and the safety threshold.

7. The infusion device of Claim 1, wherein the processor is further configured to:
determine an estimated response time for the alarm based on historical response times for the type of the alarm and the care area at which the infusion device is located; and
provide the notification only when the response time is within the lead time threshold, otherwise forgo providing the notification.

8. The infusion device of Claim 1, wherein providing the notification comprises:
identifying a clinician associated with the infusion device; and
sending a notification to a mobile device associated with the clinician.

9. The infusion device of Claim 1, wherein the alarm comprises an air-in-line alarm, an occlusion alarm, an end-of-travel alarm, or an end-of-infusion alarm.

10. A computer-implemented method for managing alarms of an infusion device, the method comprising:
monitoring sensor data generated by a sensor of an infusion device, the sensor being configured to generate the sensor data for an infusion metric associated with an infusion therapy;
determining, based on the sensor data, a predicted alarm time at which the infusion metric is predicted to satisfy a safety threshold, wherein an alarm of the infusion device is configured to trigger when the sensor data indicates that the infusion metric satisfies the safety threshold;
determining, before the predicted alarm time, a lead time threshold based on (i) a type of the alarm, (ii) a type of a fluid administered by an infusion pump of the infusion device in accordance with the infusion therapy, or (iii) a care area at which the infusion device is located, and further based on (iv) collecting historical data for clinician response times to notifications provided using an initial lead time threshold, (v) providing the historical data to a machine-learning model, (vi) receiving a new lead time threshold from the machine-learning model responsive to providing the historical data, and (vii) revising the initial lead time threshold based on the new lead time threshold; and
based on a current time being within the lead time threshold, and prior to the predicted alarm time and without triggering the alarm at the infusion device, providing a notification regarding the alarm.

11. The computer-implemented method of Claim 10, further comprising, responsive to a user interacting with the notification:
adjusting the safety threshold for a predetermined amount of time, such that the alarm will not trigger prior to or at the predicted alarm time; or
disabling the alarm for the predetermined amount of time, such that the alarm will not trigger when the sensor data indicates that the infusion metric satisfies the safety threshold.

12. The computer-implemented method of Claim 10, further comprising, responsive to a user logging into the infusion device and after determining the predicted alarm time:
providing the notification for display via a display device of the infusion device;
prompting the user to indicate whether to disable the alarm; and
responsive to receiving an indication from the user to disable the alarm, disabling the alarm for a predetermined amount of time.

13. The computer-implemented method of Claim 10, further comprising:
determining an estimated response time for the alarm based on historical response times for the type of the alarm and the care area at which the infusion device is located; and
providing the notification only when the response time is within the lead time threshold, otherwise forgoing providing the notification.

14. A non-transitory, machine-readable storage medium embodying instructions that, when executed by a machine, facilitate the machine to perform the computer-implemented method of any one of Claims 10-13.

## Patentansprüche

1. Infusionsvorrichtung, umfassend:
eine Infusionspumpe, die dazu eingerichtet ist, ein Fluid gemäß einer Infusionstherapie abzugeben;
einen Sensor, der dazu eingerichtet ist, Sensordaten für einen der Infusionstherapie zugeordneten Infusionsparameter zu erzeugen;
einen Alarm, der dazu eingerichtet ist, ausgelöst zu werden, wenn die Sensordaten anzeigen, dass der Infusionsparameter einen Sicherheitsschwellenwert erfüllt;
und einen Prozessor, der dazu eingerichtet ist:
die von dem Sensor erzeugten Sensordaten zu überwachen;
basierend auf den Sensordaten einen vorhergesagten Alarmzeitpunkt zu bestimmen, zu dem der Infusionsparameter voraussichtlich den Sicherheitsschwellenwert erfüllen wird;
vor dem vorhergesagten Alarmzeitpunkt einen Vorlaufzeitschwellenwert zu bestimmen, basierend auf (i) einer Art des Alarms, (ii) einer Art des Fluids oder (iii) einem Versorgungsbereich, an dem sich die Infusionsvorrichtung befindet, und ferner basierend auf (iv) dem Erfassen historischer Daten zu Reaktionszeiten von medizinischen Fachkräften auf Benachrichtigungen, die unter Verwendung eines anfänglichen Vorlaufzeitschwellenwerts bereitgestellt wurden, (v) dem Bereitstellen der historischen Daten für ein Modell für maschinelles Lernen, (vi) dem Empfangen eines neuen Vorlaufzeitschwellenwerts von dem Modell für maschinelles Lernen in Reaktion auf das Bereitstellen der historischen Daten, und (vii) dem Ändern des anfänglichen Vorlaufzeitschwellenwerts basierend auf dem neuen Vorlaufzeitschwellenwert;
und basierend darauf, dass ein aktueller Zeitpunkt innerhalb des Vorlaufzeitschwellenwerts liegt, sowie vor dem vorhergesagten Alarmzeitpunkt und ohne Auslösen des Alarms an der Infusionsvorrichtung, eine Benachrichtigung betreffend den Alarm bereitzustellen.

2. Infusionsvorrichtung nach Anspruch 1, wobei der anfängliche Vorlaufzeitschwellenwert unter Verwendung der Art des Alarms, der Art des Fluids oder des Versorgungsbereichs, an dem sich die Infusionsvorrichtung befindet, aus einer Nachschlagetabelle abgerufen wird.

3. Infusionsvorrichtung nach Anspruch 1, wobei der Prozessor ferner dazu eingerichtet ist, in Reaktion auf eine Interaktion eines Benutzers mit der Benachrichtigung:
den Sicherheitsschwellenwert für eine vorbestimmte Zeitdauer anzupassen, derart, dass der Alarm nicht vor oder zu dem vorhergesagten Alarmzeitpunkt ausgelöst wird;
oder den Alarm für die vorbestimmte Zeitdauer zu deaktivieren, derart, dass der Alarm nicht ausgelöst wird, wenn die Sensordaten anzeigen, dass der Infusionsparameter den Sicherheitsschwellenwert erfüllt.

4. Infusionsvorrichtung nach Anspruch 1, wobei der Prozessor ferner dazu eingerichtet ist, in Reaktion darauf, dass sich ein Benutzer bei der Infusionsvorrichtung anmeldet, und nach dem Bestimmen des vorhergesagten Alarmzeitpunkts:
die Benachrichtigung zur Anzeige über eine Anzeigevorrichtung der Infusionsvorrichtung bereitzustellen;
den Benutzer aufzufordern anzugeben, ob der Alarm deaktiviert werden soll;
und in Reaktion auf das Empfangen einer Angabe von dem Benutzer, den Alarm zu deaktivieren, den Alarm für eine vorbestimmte Zeitdauer zu deaktivieren.

5. Infusionsvorrichtung nach Anspruch 3 oder 4, wobei die vorbestimmte Zeitdauer auf der Art des Alarms, der Art des Fluids oder dem Versorgungsbereich, an dem sich die Infusionsvorrichtung befindet, basiert.

6. Infusionsvorrichtung nach Anspruch 1, wobei die Infusionsvorrichtung ferner eine Leuchtanzeige umfasst und der Prozessor ferner dazu eingerichtet ist, eine Helligkeit oder eine Farbe der Leuchtanzeige basierend auf einer Differenz zwischen dem Infusionsparameter und dem Sicherheitsschwellenwert anzupassen.

7. Infusionsvorrichtung nach Anspruch 1, wobei der Prozessor ferner dazu eingerichtet ist:
eine geschätzte Reaktionszeit für den Alarm basierend auf historischen Reaktionszeiten für die Art des Alarms und den Versorgungsbereich, an dem sich die Infusionsvorrichtung befindet, zu bestimmen;
und die Benachrichtigung nur dann bereitzustellen, wenn die Reaktionszeit innerhalb des Vorlaufzeitschwellenwerts liegt, und andernfalls von einem Bereitstellen der Benachrichtigung abzusehen.

8. Infusionsvorrichtung nach Anspruch 1, wobei das Bereitstellen der Benachrichtigung umfasst:
Identifizieren einer der Infusionsvorrichtung zugeordneten medizinischen Fachkraft;
und Senden einer Benachrichtigung an ein der medizinischen Fachkraft zugeordnetes mobiles Endgerät.

9. Infusionsvorrichtung nach Anspruch 1, wobei der Alarm einen Alarm bei Luft in der Leitung, einen Okklusionsalarm, einen Endlagenalarm oder einen Alarm bei Ende der Infusion umfasst.

10. Computerimplementiertes Verfahren zum Verwalten von Alarmen einer Infusionsvorrichtung, wobei das Verfahren umfasst:
Überwachen von Sensordaten, die von einem Sensor einer Infusionsvorrichtung erzeugt werden, wobei der Sensor dazu eingerichtet ist, die Sensordaten für einen einer Infusionstherapie zugeordneten Infusionsparameter zu erzeugen;
Bestimmen, basierend auf den Sensordaten, eines vorhergesagten Alarmzeitpunkts, zu dem der Infusionsparameter voraussichtlich einen Sicherheitsschwellenwert erfüllen wird, wobei ein Alarm der Infusionsvorrichtung dazu eingerichtet ist, ausgelöst zu werden, wenn die Sensordaten anzeigen, dass der Infusionsparameter den Sicherheitsschwellenwert erfüllt;
Bestimmen, vor dem vorhergesagten Alarmzeitpunkt, eines Vorlaufzeitschwellenwerts basierend auf (i) einer Art des Alarms, (ii) einer Art eines Fluids, das durch eine Infusionspumpe der Infusionsvorrichtung gemäß der Infusionstherapie verabreicht wird, oder (iii) einem Versorgungsbereich, an dem sich die Infusionsvorrichtung befindet, und ferner basierend auf (iv) dem Erfassen historischer Daten zu Reaktionszeiten von medizinischen Fachkräften auf Benachrichtigungen, die unter Verwendung eines anfänglichen Vorlaufzeitschwellenwerts bereitgestellt wurden, (v) dem Bereitstellen der historischen Daten für ein Modell für maschinelles Lernen, (vi) dem Empfangen eines neuen Vorlaufzeitschwellenwerts von dem Modell für maschinelles Lernen in Reaktion auf das Bereitstellen der historischen Daten, und (vii) dem Ändern des anfänglichen Vorlaufzeitschwellenwerts basierend auf dem neuen Vorlaufzeitschwellenwert;
und Bereitstellen, basierend darauf, dass ein aktueller Zeitpunkt innerhalb des Vorlaufzeitschwellenwerts liegt, sowie vor dem vorhergesagten Alarmzeitpunkt und ohne Auslösen des Alarms an der Infusionsvorrichtung, einer Benachrichtigung betreffend den Alarm.

11. Computerimplementiertes Verfahren nach Anspruch 10, ferner umfassend, in Reaktion auf eine Interaktion eines Benutzers mit der Benachrichtigung:
Anpassen des Sicherheitsschwellenwerts für eine vorbestimmte Zeitdauer, derart, dass der Alarm nicht vor oder zu dem vorhergesagten Alarmzeitpunkt ausgelöst wird;
oder Deaktivieren des Alarms für die vorbestimmte Zeitdauer, derart, dass der Alarm nicht ausgelöst wird, wenn die Sensordaten anzeigen, dass der Infusionsparameter den Sicherheitsschwellenwert erfüllt.

12. Computerimplementiertes Verfahren nach Anspruch 10, ferner umfassend, in Reaktion darauf, dass sich ein Benutzer bei der Infusionsvorrichtung anmeldet, und nach dem Bestimmen des vorhergesagten Alarmzeitpunkts:
Bereitstellen der Benachrichtigung zur Anzeige über eine Anzeigevorrichtung der Infusionsvorrichtung;
Auffordern des Benutzers anzugeben, ob der Alarm deaktiviert werden soll;
und in Reaktion auf das Empfangen einer Angabe von dem Benutzer, den Alarm zu deaktivieren, Deaktivieren des Alarms für eine vorbestimmte Zeitdauer.

13. Computerimplementiertes Verfahren nach Anspruch 10, ferner umfassend:
Bestimmen einer geschätzten Reaktionszeit für den Alarm basierend auf historischen Reaktionszeiten für die Art des Alarms und den Versorgungsbereich, an dem sich die Infusionsvorrichtung befindet;
und Bereitstellen der Benachrichtigung nur dann, wenn die Reaktionszeit innerhalb des Vorlaufzeitschwellenwerts liegt, und andernfalls Absehen von einem Bereitstellen der Benachrichtigung.

14. Nichttransitorisches, maschinenlesbares Speichermedium, das Anweisungen verkörpert, die, wenn sie von einer Maschine ausgeführt werden, es der Maschine erleichtern, das computerimplementierte Verfahren nach einem der Ansprüche 10 bis 13 durchzuführen.

## Revendications

1. Dispositif de perfusion, comprenant :
une pompe à perfusion configurée pour délivrer un fluide conformément à une thérapie par perfusion ;
un capteur configuré pour générer des données de capteur pour un paramètre de perfusion associé à la thérapie par perfusion ;
une alarme configurée pour se déclencher lorsque les données de capteur indiquent que le paramètre de perfusion satisfait un seuil de sécurité ;
et un processeur configuré pour :
surveiller les données de capteur générées par le capteur ;
déterminer, sur la base des données de capteur, un instant d'alarme prédit auquel, selon la prédiction, le paramètre de perfusion satisfera le seuil de sécurité ;
déterminer, avant l'instant d'alarme prédit, un seuil de délai d'anticipation sur la base (i) d'un type de l'alarme, (ii) d'un type du fluide, ou (iii) d'une zone de soins où se trouve le dispositif de perfusion, et en outre sur la base (iv) de la collecte de données historiques relatives aux temps de réponse des professionnels de santé à des notifications fournies en utilisant un seuil de délai d'anticipation initial, (v) de la fourniture des données historiques à un modèle d'apprentissage automatique, (vi) de la réception d'un nouveau seuil de délai d'anticipation du modèle d'apprentissage automatique en réponse à la fourniture des données historiques, et (vii) de la révision du seuil de délai d'anticipation initial sur la base du nouveau seuil de délai d'anticipation ;
et sur la base du fait qu'un instant actuel se situe dans le seuil de délai d'anticipation, et avant l'instant d'alarme prédit et sans déclencher l'alarme au niveau du dispositif de perfusion, fournir une notification concernant l'alarme.

2. Dispositif de perfusion selon la revendication 1, dans lequel le seuil de délai d'anticipation initial est extrait d'une table de consultation en utilisant le type de l'alarme, le type du fluide, ou la zone de soins où se trouve le dispositif de perfusion.

3. Dispositif de perfusion selon la revendication 1, dans lequel le processeur est en outre configuré pour, en réponse à une interaction d'un utilisateur avec la notification :
ajuster le seuil de sécurité pendant une durée prédéterminée, de sorte que l'alarme ne se déclenche pas avant ou à l'instant d'alarme prédit ;
ou désactiver l'alarme pendant la durée prédéterminée, de sorte que l'alarme ne se déclenche pas lorsque les données de capteur indiquent que le paramètre de perfusion satisfait le seuil de sécurité.

4. Dispositif de perfusion selon la revendication 1, dans lequel le processeur est en outre configuré pour, en réponse au fait qu'un utilisateur se connecte au dispositif de perfusion et après avoir déterminé l'instant d'alarme prédit :
fournir la notification pour affichage par l'intermédiaire d'un dispositif d'affichage du dispositif de perfusion ;
inviter l'utilisateur à indiquer si l'alarme doit être désactivée ;
et en réponse à la réception d'une indication de la part de l'utilisateur de désactiver l'alarme, désactiver l'alarme pendant une durée prédéterminée.

5. Dispositif de perfusion selon la revendication 3 ou 4, dans lequel la durée prédéterminée est basée sur le type de l'alarme, le type du fluide, ou la zone de soins où se trouve le dispositif de perfusion.

6. Dispositif de perfusion selon la revendication 1, dans lequel le dispositif de perfusion comprend en outre un indicateur lumineux, et le processeur est en outre configuré pour ajuster une luminosité ou une couleur de l'indicateur lumineux sur la base d'une différence entre le paramètre de perfusion et le seuil de sécurité.

7. Dispositif de perfusion selon la revendication 1, dans lequel le processeur est en outre configuré pour :
déterminer un temps de réponse estimé pour l'alarme sur la base de temps de réponse historiques pour le type de l'alarme et la zone de soins où se trouve le dispositif de perfusion ;
et fournir la notification uniquement lorsque le temps de réponse se situe dans le seuil de délai d'anticipation, et sinon s'abstenir de fournir la notification.

8. Dispositif de perfusion selon la revendication 1, dans lequel la fourniture de la notification comprend :
l'identification d'un professionnel de santé associé au dispositif de perfusion ;
et l'envoi d'une notification à un terminal mobile associé au professionnel de santé.

9. Dispositif de perfusion selon la revendication 1, dans lequel l'alarme comprend une alarme de présence d'air dans la ligne, une alarme d'occlusion, une alarme de fin de course, ou une alarme de fin de perfusion.

10. Procédé mis en œuvre par ordinateur pour gérer des alarmes d'un dispositif de perfusion, le procédé comprenant :
la surveillance de données de capteur générées par un capteur d'un dispositif de perfusion, le capteur étant configuré pour générer les données de capteur pour un paramètre de perfusion associé à une thérapie par perfusion ;
la détermination, sur la base des données de capteur, d'un instant d'alarme prédit auquel, selon la prédiction, le paramètre de perfusion satisfera un seuil de sécurité, dans lequel une alarme du dispositif de perfusion est configurée pour se déclencher lorsque les données de capteur indiquent que le paramètre de perfusion satisfait le seuil de sécurité ;
la détermination, avant l'instant d'alarme prédit, d'un seuil de délai d'anticipation sur la base (i) d'un type de l'alarme, (ii) d'un type d'un fluide administré par une pompe à perfusion du dispositif de perfusion conformément à la thérapie par perfusion, ou (iii) d'une zone de soins où se trouve le dispositif de perfusion, et en outre sur la base (iv) de la collecte de données historiques relatives aux temps de réponse des professionnels de santé à des notifications fournies en utilisant un seuil de délai d'anticipation initial, (v) de la fourniture des données historiques à un modèle d'apprentissage automatique, (vi) de la réception d'un nouveau seuil de délai d'anticipation du modèle d'apprentissage automatique en réponse à la fourniture des données historiques, et (vii) de la révision du seuil de délai d'anticipation initial sur la base du nouveau seuil de délai d'anticipation ;
et sur la base du fait qu'un instant actuel se situe dans le seuil de délai d'anticipation, et avant l'instant d'alarme prédit et sans déclencher l'alarme au niveau du dispositif de perfusion, la fourniture d'une notification concernant l'alarme.

11. Procédé mis en œuvre par ordinateur selon la revendication 10, comprenant en outre, en réponse à une interaction d'un utilisateur avec la notification :
l'ajustement du seuil de sécurité pendant une durée prédéterminée, de sorte que l'alarme ne se déclenche pas avant ou à l'instant d'alarme prédit ;
ou la désactivation de l'alarme pendant la durée prédéterminée, de sorte que l'alarme ne se déclenche pas lorsque les données de capteur indiquent que le paramètre de perfusion satisfait le seuil de sécurité.

12. Procédé mis en œuvre par ordinateur selon la revendication 10, comprenant en outre, en réponse au fait qu'un utilisateur se connecte au dispositif de perfusion et après avoir déterminé l'instant d'alarme prédit :
la fourniture de la notification pour affichage par l'intermédiaire d'un dispositif d'affichage du dispositif de perfusion ;
l'invitation de l'utilisateur à indiquer si l'alarme doit être désactivée ;
et en réponse à la réception d'une indication de la part de l'utilisateur de désactiver l'alarme, la désactivation de l'alarme pendant une durée prédéterminée.

13. Procédé mis en œuvre par ordinateur selon la revendication 10, comprenant en outre :
la détermination d'un temps de réponse estimé pour l'alarme sur la base de temps de réponse historiques pour le type de l'alarme et la zone de soins où se trouve le dispositif de perfusion ;
et la fourniture de la notification uniquement lorsque le temps de réponse se situe dans le seuil de délai d'anticipation, et sinon l'abstention de fournir la notification.

14. Support de stockage non transitoire, lisible par machine, incorporant des instructions qui, lorsqu'elles sont exécutées par une machine, facilitent la mise en œuvre, par la machine, du procédé mis en œuvre par ordinateur selon l'une quelconque des revendications 10 à 13.
